# FASCICULE DE BREVET EUROPEEN

(11) **EP 0 716 594 B1**
(45) Date de publication et mention de la délivrance du brevet: **04.10.2001**
(21) Numéro de dépôt: 95923427.9
(22) Date de dépôt: 22.06.1995
(51) Int. Cl.: A61K 7/48, A61K 7/06

(54) **UNE COMPOSITION TOPIQUE COMPRENANT UN AGENT EPAISSISSANT**
VERDICKUNGSMITTEL ENTHALTENDE TOPISCHE ZUSAMMENSETZUNG
TOPICAL COMPOSITION INCLUDING A THICKENING AGENT

(30) Priorité: 22.06.1994 FR 9407636
(43) Date de publication de la demande: 19.06.1996
(73) Titulaire: S.E.P.P.I.C., SOCIETE D'EXPLOITATION DE PRODUITS POUR LES INDUSTRIES CHIMIQUES, F-75007 Paris (FR)
(72) Inventeur: MICHEL-LECOCU, Nelly, F-94700 Maisons-Alfort (FR); AMALRIC, Chantal, F-81700 Blan (FR)
(74) Mandataire: Le Moenner, Gabriel
(86) Numéro de dépôt international: FR9500833
(87) Numéro de publication internationale: WO9535089

(56) Documents cités:
- EP-A- 0 186 361
- EP-A- 0 424 260
- EP-A- 0 494 022
- WO-A-92/21316

## Description

La présente invention concerne, d'une part, une composition topique, utile notamment en cosmétique et en dermopharmacie, comprenant une phase aqueuse et un agent épaississant, et d'autre part un nouvel agent épaississant en tant que tel, présentant une bonne tolérance vis-à-vis de la peau et des muqueuses.

La demande de brevet EP-A-0.424.260 et la demande internationale WO 92/21316, décrivent des compositions cosmétiques ou de traitement des matières kératiniques pouvant comprendre un copolymère d'acrylate d'ammonium/acrylamide dispersé dans une émulsion eau dans huile. Cette dispersion comprend des tensioactifs oléosolubles ayant un rapport hydrophile/lipophile (ou HLB) inférieur à 7. Elle est utile en tant qu'agent épaississant. La Demanderesse a cependant pu constater que ce type d'agent épaississant, se présentant sous forme d'une dispersion, convient mal pour la préparation de certaines compositions, telles des émulsions comprenant au moins une phase huile et au moins une phase aqueuse. En effet, ce type d'agent épaississant est difficilement miscible avec les huiles polaires pouvant constituer la phase huile de ces émulsions.

La demande de brevet EP-A-0.503.853 décrit des compositions topiques, c'est-à-dire des compositions destinées à être appliquées sur la peau ou les muqueuses de l'homme ou de l'animal, comprenant un agent épaississant constitué d'une émulsion inverse eau dans huile et d'un copolymère. Le copolymère est essentiellement en solution dans la phase aqueuse constituant ladite émulsion inverse. Le copolymère est constitué d'unités dérivées (a) d'un monomère monoacrylamide, (b) d'un monomère d'acide 2-acrylamido-2-méthyl-propane-sulfonique (AMPS) et (c) d'un monomère polyfonctionnel. Un tel agent épaississant a pour avantage de pouvoir s'inverser lorsqu'il est dilué dans une phase hydrophile. Ainsi, lorsque l'agent épaississant qui comprend une émulsion inverse eau dans huile est mélangé aux autres composants formant ladite composition topique, le sens de l'émulsion s'inverse, de sorte que la phase aqueuse devient continue. Cet agent épaississant convient parfaitement pour la préparation de compositions topiques comprenant notamment des huiles polaires avec lesquels il est parfaitement miscible. L'agent épaississant l'avantage d'être relativement bien toléré par les tissus cutanés et les muqueuses sur lesquels il est appliqué.

Toutefois, la Demanderesse a pu constater que les agents épaississants mis en oeuvre dans les compositions topiques selon la demande de brevet EP-A-0.503.853 ne pouvaient être convenablement utilisés pour la fabrication de tous types de compositions topiques. Ainsi, il peut être difficile de préparer des émulsions fluides, épaissies par ce type d'agents épaississants, ce de manière reproductible en termes de viscosité. On entend ici par "émulsions fluides" des compositions topiques présentant une viscosité inférieure à environ 25 Pa.s (Brookfield, LV4, 6 tours/minute). En effet, ce type de compositions topiques requiert des teneurs relativement faibles en ledit agent épaississant. Or, en raison de la nature de ce dernier, de légères variations dans les concentrations en agents épaississants mis en oeuvre peuvent conduire à d'importantes variations de la viscosité de la composition topique. Il est alors difficile à l'échelle industrielle de préparer des compositions topiques avec un tel agent épaississant de manière reproductible.

Il a également été constaté que l'effet viscosant procuré par l'agent épaississant décrit dans ce document est réduit lorsque cet agent, épaississant est associé dans la composition topique à des électrolytes, tels le chlorure de sodium ou le chlorure de magnésium.

Par ailleurs, la demande de brevet EP-A-0.186.361, décrit des agents épaississants utiles pour préparer des encres d'impression pâteuses. L'agent épaississant consiste notamment en une émulsion eau dans huile comprenant une phase aqueuse dans laquelle est dissous un homopolymère ou un copolymère à base, préférentiellement, de motifs dérivés de l'acide acrylique. Cet agent épaississant, tout comme l'agent épaississant décrit dans EP-A-0.503.853, est préparé selon le procédé connu en lui-même de polymérisation en émulsion inverse. Selon ce procédé, la polymérisation des monomères est réalisée au sein d'une émulsion eau dans huile. La phase huile constitutive de cette émulsion est habituellement à base d'une huile volatile, telle une huile d'isoparaffine. La demanderesse a cependant constaté que cet agent épaississant n'était pas bien toléré par la peau.

Elle a alors mis au point une composition topique, obviant les inconvénients des compositions topiques mentionnés plus haut. Plus particulièrement, la composition topique selon l'invention comprend un agent épaississant à base d'une émulsion inverse, bien tolérée par les tissus sur lesquels elle est appliquée, et qui de plus, présente une viscosité stable même en présence d'électrolytes.

Selon un autre aspect, l'invention concerne un agent épaississant comprenant un copolymère, pouvant être facilement utilisé pour épaissir et émulsionner une composition topique, comprenant une émulsion inverse, ledit agent épaississant permettant l'obtention d'une très bonne tolérance vis-à-vis des tissus sur lesquels il est appliqué.

L'agent épaississant consiste en une émulsion eau dans huile, dans laquelle la phase aqueuse comprend un copolymère ayant des motifs dérivés (i) de l'acide acrylique sous forme libre ou sous forme de sel de sodium et (ii) d'un monomère monoacrylamide, caractérisé en ce que :
a) La phase huile est constituée d'un mélange dans un rapport pondéral compris entre 70/30 et 30/70, d'au moins une huile volatile choisie parmi les isoparaffines et d'au moins une huile non volatile choisie parmi les huiles de paraffine, les esters comportant plus de 10 atomes de carbone et, de préférence, de 14 à 34 atomes de carbone, formés par la condensation entre un acide gras et un monoalcool, et
b) le rapport molaire entre le monomère mono carboxylique et le monomère acrylamide, est compris entre 85/15 et 15/85 et de préférence entre 60/40 et 40/60.

Dans le cadre de la présente invention, afin de déterminer si une huile est volatile, on procède au test suivant : on introduit 3 g de l'huile à considérer dans un récipient de 250 ml que l'on porte à 220°C pendant 1 H 30. La perte de poids est alors mesurée. Une huile est considérée comme volatile si dans ces conditions, le pourcentage de perte de poids est supérieur à 5 %.

Les isoparaffines volatiles pouvant être mises en oeuvre dans le cadre de la présente invention, présentent une structure d'hydrocarbure ramifié saturé et un poids moléculaire inférieur à environ 200. A titre d'huiles d'isoparaffine, on peut plus particulièrement citer l'ISOPAR M et l'ISOPAR L, commercialisés par la société EXXON.

Les huiles de paraffine non volatiles pouvant être mises en oeuvre dans le cadre de la présente invention, sont les huiles de paraffine épaisses ou légères, telles que définies dans la pharmacopée européenne. Comme esters formés par la condensation entre un acide gras et un monoalcool, ledit ester comportant plus de 10 atomes de carbone, de préférence de 14 à 34 atomes de carbone, il y a le cétéaryloctanoate ou l'isostéaryl isostéarate.

Selon un aspect avantageux de l'invention, l'huile volatile est une huile d'isoparaffine et l'huile non volatile comprend au moins une huile de paraffine ou l'isostéaryl isostéarate .

Habituellement, le copolymère compris dans l'agent épaississant défini précédemment, comporte en outre un monomère polyfonctionnel. Celui-ci peut consister en le méthylène bis-acrylamide (MBA), le diallylphtalate, les diacrylateglycols, le cyanurate de triallyle, l'isocyanurate de triallyle, les alkylène-éthers polyfonctionnels, l'allyl méthacrylate, ou des mélanges de l'un ou plusieurs de ces monomères polyfonctionnels. Un monomère polyfonctionnel préféré dans le cadre de la présente invention consiste en le MBA.

La teneur en monomères polyfonctionnels présents dans le copolymère est comprise entre 0,1 et 2 milli-équivalents/mole en les autres monomères.

L'agent épaississant peut en outre avantageusement comporter un ou plusieurs agents tensioactifs. Ces tensioactifs sont de préférence du type non ionique, tels que les alcools gras polyéthoxylés, les alkylphénols polyéthoxylés, comme le nonylphénol polyéthoxylé, les esters de sorbitan polyéthoxylés, les esters de sorbitan, les alkylolamides ou les alkylpolyglycosides, notamment ceux définis dans les demandes de brevet EP-A-0.077.167 ou EP-A-0.358.216, dont référence est intégrée à la présente description.

Selon un aspect tout particulièrement avantageux de la présente invention, l'agent épaississant mis en oeuvre comprend un ou plusieurs agents tensioactifs dont la HLB est supérieure ou égale à 8, de préférence supérieure ou égale à 10.

Un agent épaississant selon l'invention peut être préparé selon le procédé décrit dans la demande de brevet EP-A-0.186.361, dont référence est intégrée à la présente description, en particulier les passages page 7, lignes 1-9 et page 10, lignes 22-37, ainsi que les exemples 1, 3 et 5.

Selon ce procédé, les différents monomères constitutifs du copolymère sont mis en solution aqueuse. Cette solution aqueuse est ensuite mélangée avec une phase huile comprenant un ou plusieurs agents tensioactifs, notamment des agents tensioactifs non ioniques. Ces agents tensioactifs présentent une valeur de HLB inférieure à 9. La solution aqueuse et la phase huile forment ainsi une émulsion eau dans huile. Après quoi, on procède à la copolymérisation des monomères de la phase aqueuse en utilisant, de manière classique des monomères polyfonctionnels tels que ceux mentionnés plus haut. L'huile constitutive de la phase huile de ladite émulsion inverse eau dans huile est habituellement constituée d'une ou plusieurs huiles volatiles.

l'huile non volatile que l'agent épaississant conforme à l'invention comporte, peut être incorporée dans la phase huile de l'émulsion eau dans huile après copolymérisation. On peut également rajouter après copolymérisation des tensioactifs non ioniques, tels ceux cités plus haut, en vue d'obtenir une HLB supérieure ou égale à 8.

Selon un aspect avantageux de la présente invention, l'agent épaississant mis en oeuvre comprend une émulsion eau dans huile comportant de 10 à 60 % en poids de phase huile, de 30 à 70 % en poids d'eau, de 10 à 30 % en poids d'un copolymère et de 5 à 20 % en poids d'agent tensioactif.

Selon un autre aspect de la présente invention celle-ci a pour objet, l'utilisation de l'agent épaississant tel que défini précédemment, pour la préparation d'une composition topique.

Selon encore un autre aspect, l'invention concerne des compositions topiques, notamment des émulsions fluides, comprenant l'agent épaississant, ladite composition topique pouvant être aisément préparée de manière reproductible à l'échelle industrielle.

Une composition topique comprend au moins une phase aqueuse, une huile non volatile et un agent épaississant tel que défini ci-dessus.

Il est bien entendu que, dans le cadre de la présente invention, le sens de l'émulsion constitutive de l'agent épaississant, est le sens existant préalablement aux mélanges de cet agent épaississant avec les autres composants constitutifs de la composition topique, en particulier les composants hydrophiles. Ainsi, il est notable que l'agent épaississant, conforme à l'invention, comprenant une émulsion inverse eau dans huile, peut s'inverser pour former une émulsion dont la phase continue est une phase aqueuse, cette inversion se produisant lors de la dilution de l'agent épaississant avec une phase hydrophile constitutive de la composition topique. Après inversion, le copolymère est libéré dans la phase continue et lui confère ainsi une viscosité accrue.

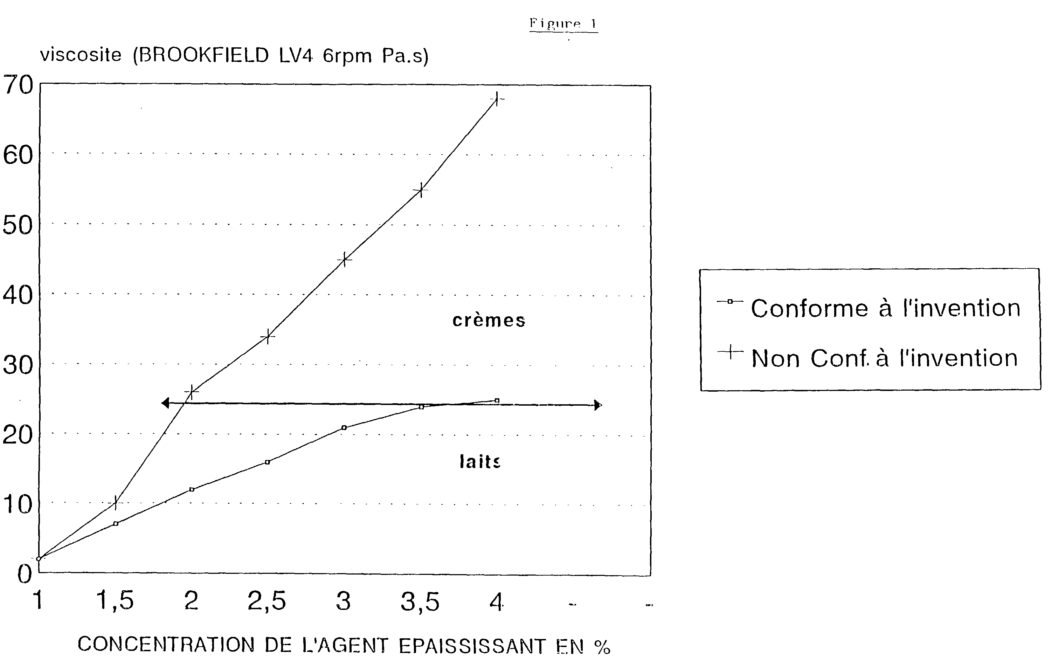

D'autres aspects de la présente invention ressortiront de la description suivante et de la figure.

La figure représente la viscosité, mesurée par un viscosimètre Brookfield LV4, 6 tours/minute, d'une composition topique obtenue au moyen de concentrations croissantes d'un agent épaississant conforme à l'invention, comparée à la viscosité d'une composition topique similaire de l'art antérieur, comprenant également un agent épaississant.

La demanderesse a constaté qu'une composition topique comprenant une huile non volatile ainsi qu'un agent épaississant, tel que décrit plus haut, était parfaitement tolérée par les tissus sur lesquels elle était appliquée.

L'huile non volatile présente dans la composition topique peut être constitutive, en partie au moins, de la phase huile de l'émulsion inverse formant l'agent épaississant. Cette phase huile est alors essentiellement constituée d'un mélange d'au moins une huile volatile et d'au moins une huile non volatile.

Habituellement le rapport pondéral entre l'huile non volatile et l'huile volatile au sein de la composition topique conforme à l'invention, est supérieur à 0,5, de préférence supérieur à 0,9 et plus généralement compris entre 1 et 100.

La composition topique selon l'invention, destinée à être appliquée sur la peau ou les muqueuses de l'homme ou de l'animal, consiste en une émulsion topique comprenant au moins une phase aqueuse et au moins une phase huile, du type huile dans eau. Plus particulièrement, cette émulsion topique peut consister en une émulsion fluide, telle un lait ou un gel fluide. La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau et des muqueuses. Dans ce dernier cas, la composition topique comporte alors un principe actif qui peut par exemple consister en un agent anti-inflammatoire, un myorelaxant, un antifongique ou un antibactérien. Lorsque la composition topique est utilisée en tant que composition cosmétique destinée à être appliquée sur la peau ou les muqueuses, elle peut ou non comporter un principe actif, par exemple un agent hydratant, un agent bronzant, un filtre solaire, un antirides, un agent à visée amincissante, un agent antiradicalaire, un agent antiacnéique ou un antifongique.

Une composition topique selon l'invention comporte entre 0,1 et 10 % en poids de l'agent épaississant défini ci-dessus. Le pH de la composition topique est de préférence supérieur ou égal à 5, plus préférentiellement, il est compris entre 6 et 12.

La composition topique peut en outre comporter des composés classiquement compris dans ce type de compositions, par exemple des parfums, des conservateurs, des colorants, des émollients ou des tensioactifs.

Une composition topique selon l'invention peut être préparée de manière connue de l'homme du métier, par exemple selon le procédé décrit dans la demande de brevet EP-A-0.503.853.

Selon encore un autre aspect, l'invention concerne l'utilisation du nouvel agent épaississant conforme à l'invention, mentionné ci-dessus, pour épaissir et émulsionner une composition topique comprenant au moins une phase aqueuse.

Les exemples qui suivent ont pour but d'illustrer la présente invention.

### Exemple 1 :

On prépare un agent épaississant conforme à la présente invention, en mettant en oeuvre le procédé décrit dans la demande de brevet EP-A-0.186.361, de sorte à obtenir une émulsion eau dans huile présentant la composition suivante (% en poids) :
- copolymère comprenant :
   * des motifs dérivés d'un monomère monoacrylamide 7,5 %
   * motifs dérivés d'un monomère d'acrylate de sodium 9,5 %
   * N,N-méthylène bis acrylamide 0,2 meq/mole de monomère
- isoparaffine 15 %
- huile de paraffine 15 %
- MONTANOX 85 SPI ⁽¹⁾ 4 %
- Laureth 7⁽²⁾ 2%
- eau q.s. 100 %

⁽¹⁾ : polysorbate commercialisé par la société SEPPIC
⁽²⁾ : alcool laurique éthoxylé par 7 motifs d'oxyde d'éthylène.

La copolymérisation des monomères est réalisée selon le procédé de polymérisation en émulsion inverse, au sein d'une émulsion eau dans huile, dont la phase huile est constituée par l'isoparaffine. L'huile de paraffine est introduite dans le mélange obtenu après copolymérisation.

### Exemple 2 :

On prépare un autre agent épaississant conforme à la présente invention selon le procédé décrit dans la demande de brevet EP-A-0.186.361. A cet effet, dans un premier temps, on prépare une émulsion eau dans huile comprenant :
- copolymère comprenant des motifs dérivés :
   * d'un monomère monoacrylamide 8,3 %
   * d'un monomère acrylate de sodium 10,8 %
   * N,N-méthylène bis acrylamide 0,2 meq/mole de monomère
- isoparaffine 15 %
- MONTANE 80⁽³⁾ 1,2 %
- oléamide DEA⁽⁴⁾ 2,4 %
- eau 38,3 %
puis, dans une deuxième étape, on introduit dans cette émulsion, un mélange comprenant 15 % en poids d'isostéarate d'isostéaryle et 9 % en poids de MONTANOX 60⁽⁵⁾ (par rapport au poids total de l'émulsion finale obtenue).
⁽³⁾ : oléate de sorbitan commercialisé par la société SEPPIC
⁽⁴⁾ : mélange à base de diéthanolamide d'acide oléique (nomenclature INCI)
⁽⁵⁾ : stéarate de sorbitan éthoxylé par 20 motifs d'oxyde d'éthylène (ou Polysorbate 60), commercialisé par la société SEPPIC.

### Exemple 3 :

On procède comme dans l'exemple 1 en mettant en oeuvre 12 % en poids de monomère monoacrylamide et 6 % en poids d'un monomère acrylate de sodium, de manière à obtenir un autre agent épaississant conforme à l'invention.

### Exemple 4 :

On prépare une émulsion huile dans eau en mélangeant les composés suivants (% en poids) :
- agent épaississant x %
- PRIMOL 352 ⁽⁶⁾ 10 %
- eau q.s. 100%

⁽⁶⁾ : huile de paraffine commercialisée par la société ESSO.

L'agent épaississant mis en oeuvre est soit celui de l'exemple 1, conforme à l'invention, soit un agent épaississant comprenant le copolymère tel que préparé dans l'exemple 1 de la demande de brevet EP-A-0.503.853, à titre comparatif. L'agent épaississant comparatif est préparé selon le procédé de l'exemple 1 de EP-A-0.503.853, après adaptation en vue d'obtenir une composition ne différant de celle de l'agent épaississant conforme à l'invention que par la nature même du copolymère. Cette adaptation consiste à diluer l'agent épaississant selon l'exemple 1 de EP-A-0.503.853 avec de l'eau et des huiles adéquates, afin que la concentration en copolymères soit identique à celle du copolymère de l'agent épaississant selon l'exemple 1 ci-dessus.

La phase huile, la phase aqueuse et les différents agents tensioactifs sont donc identiques quantitativement et qualitativement pour chaque agent épaississant testé. De plus, chacun de ces agents épaississants comporte une concentration identique en copolymères.

On mesure la viscosité de différentes émulsions huile dans eau telles que définies ci-dessus, comprenant des concentrations variables en agent épaississant conforme à l'invention ou en agent épaississant selon l'art antérieur.

La viscosité mesurée est exprimée en Pa.s. Elle est mesurée au moyen d'un viscosimètre Brookfield LV4, à 6 tours/minute. Les résultats obtenus sont présentés sur la figure. La viscosité est en ordonnée. La concentration en agent épaississant (% en poids) est en abscisse.

On considère qu'en dessous d'une viscosité de 25 Pa.s environ, l'émulsion huile dans l'eau est un lait (émulsion fluide) et qu'au-dessus de cette valeur, elle se présente sous la forme d'une crème.

On peut constater que dès que la concentration en l'agent épaississant selon l'art antérieur dépasse 2 % en poids, ladite émulsion se présente sous la forme d'une crème. Au contraire, l'agent épaississant conforme à l'invention peut être mis en oeuvre à des concentrations supérieures à 3 % en poids, sans que l'émulsion se présente sous forme d'une crème.

La figure montre encore que la courbe conforme à l'invention présente une pente beaucoup plus faible que celle obtenue avec un agent épaississant selon l'art antérieur. Il en ressort que de légères variations de concentrations en l'agent épaississant selon l'invention ne modifient pas de manière conséquente la viscosité de la composition préparée. Au contraire, de légères variations en la concentration de l'agent épaississant selon l'art antérieur conduisent immédiatement à des modifications importantes de la viscosité de la composition préparée.

L'agent épaississant conforme à l'invention permet donc l'obtention d'émulsions fluides, telles un lait, de manière reproductible à l'échelle industrielle, ce qui n'est pas le cas de l'agent épaississant de l'art antérieur.

### Exemple 5 :

On a testé la tolérance cutanée des agents épaississants des exemples 1 et 3. L'huile non volatile constitutive de l'agent épaississant, introduite après copolymérisation, est de nature variable.

On a également fait varier les rapports en poids entre l'isoparaffine et l'huile non volatile, constitutives de l'agent épaississant.

Les tests ont été réalisés à partir d'une solution aqueuse comprenant (% en poids) :
- agent épaississant 5,0 %
- eau 95 %

Les résultats obtenus figurent dans le tableau ci-dessous.

| Tests | Huile non volatile | Poids moléculaire de l'huile non volatile | Tolérance (%) en fonction du rapport pondéral isoparaffine/huile non volatile | | |
|---|---|---|---|---|---|
| | | | 47/53 | 67/33 | 100/0 |
| 1 | PRIMOL 352⁽¹⁾ | 470 | 100 | 60 | 15 |
| 2 | Isostéaryl d'isostéarate | 536 | 85 | | |

| | | | | | |
|---|---|---|---|---|---|
| ⁽¹⁾: Huile de paraffine épaisse commercialisée par la société ESSO. | | | | | |

La tolérance cutanée a été déterminée selon la technique dite du test épicutané sous occlusion (patch test) sur l'homme.

On a utilisé des cupules d'aluminium de 8 mm de diamètre et de 20 microlitres de capacité, chaque cupule permettant de couvrir une surface de 50 mm². Elles sont montées par paire sur un ruban adhésif.

La solution aqueuse imprègne des disques de papier-buvard, spécialement adaptés au système cupule/adhésif. Le système est appliqué sur la peau (région sous-capulaire gauche) des sujets. Quatorze volontaires, dont la moyenne d'âge est de 29 ans, ont servi de sujets.

A titre de référence, on a également appliqué de la même façon sur chaque sujet mais sur une zone de peau différente, une solution à 2 % de laurylsulfate de sodium, ainsi que de l'eau distillée servant de témoin.

24 heures après l'application, les cupules sont retirées des zones sur lesquelles elles ont été appliquées.

La lecture des résultats est réalisée 30 minutes, puis 24 heures après la dépose.

Afin d'examiner si les produits appliqués sont tolérés ou non, on a considéré l'apparition des phénomènes suivants :
- érythème,
- oedème,
- vésicule,
- sécheresse cutanée,
- rugosité cutanée,
- réflectivité de la peau.

Le pourcentage de tolérance cutanée exprimé dans le tableau ci-dessus correspond au nombre de sujets n'ayant présenté aucun phénomène mentionné ci-dessus, par rapport au nombre total des sujets, à la lecture de 24 heures.

### Exemple 6 :

En vue d'apprécier la stabilité de la viscosité conférée par l'agent épaississant selon l'invention, on a préparé une dispersion dans l'eau en l'agent épaississant selon l'exemple 1, de sorte que la viscosité obtenue soit de 80 Pa.s (Brookfield LVT4, 6 tours/minute).

A titre comparatif, on a préparé une dispersion aqueuse épaissie à 80 Pa.s au moyen de l'agent épaississant comparatif défini dans l'exemple 3.

Dans chacune des dispersions aqueuses épaissies, on a alors introduit 0,1 % en poids d'un sel. Ce sel est soit le chlorure de sodium, soit le chlorure de magnésium (MgCl₂).

La dispersion aqueuse épaissie avec l'agent épaississant conforme à l'invention dans laquelle on rajoute le chlorure de sodium voit alors sa viscosité réduite à 57 Pa.s avec NaCI et à 61 Pa.s avec MgCl₂. La dispersion aqueuse épaissie avec l'agent épaississant comparatif voit sa viscosité chuter, avec le chlorure de sodium à 13 Pa.s et avec le chlorure de magnésium à 16 Pa.s.

### Exemple 7 :

On prépare un lait démaquillant en mélangeant (% en poids) :
- MONTANOV 94⁽⁷⁾ : 3,0 %
- PRIMOL 352 : 8,0 %
- HAD⁽⁸⁾ : 2,0 %
- agent épaississant de l'exemple 1 : 0,8 %
- eau q.s. 100%

⁽⁷⁾ : tensioactif non ionique commercialisé par la société SEPPIC
⁽⁸⁾ : huile d'amande douce commercialisée par la société BERTIN.

A ce mélange, on ajoute un conservateur et un parfum.

La viscosité de cette composition est de 15 Pa.s et son pH est de 7 (après ajustement) ; sa stabilité est de 1 mois à 50°C et de 3 mois à 40°C.

### Exemple 8 :

On prépare un lait corporel en mélangeant à froid (% en poids) :
- LANOL 99⁽⁹⁾ : 10,0 %
- agent épaississant de l'exemple 1 : 1,4 %
- MICROPEARL M100⁽¹⁰⁾ : 0,2 %
- eau q.s. 100%

⁽⁹⁾ : isononyl isononanoate commercialisé par la société SEPPIC
⁽¹⁰⁾: polyméthylméthacrylate commercialisé par la société SEPPIC

A ce mélange, on ajoute un conservateur et un parfum.

La viscosité de cette composition est de 12 Pa.s et son pH est de 6 (après ajustement) ; sa stabilité est de 2 semaines à 50°C et de 3 mois à 40°C.

### Exemple 9 :

On prépare un lait corporel en mélangeant (% en poids) :
- MONTANOV 94 : 3,5 %
- LANOL 37T⁽¹¹⁾ : 8,0%
- benzophénone : 2,0 %
- diméthicone 350 cPs. : 0,05 %
- SOLAGUM L⁽¹²⁾ : 0,05 %
- agent épaississant de l'exemple 1 : 1,5 %
- eau q.s. 100%

⁽¹¹⁾ : triheptanoate de glycérol commercialisé par la société SEPPIC
⁽¹²⁾ : carréghénane commercialisé par la société SEPPIC

A ce mélange, on ajoute un conservateur et un parfum.

La viscosité de cette composition est de 18 Pa.s et son pH est de 6 (après ajustement) ; sa stabilité est de 1 mois à 50°C et de 3 mois à 40°C.

### Exemple 10 :

On prépare une émulsion fluide à pH alcalin, en mélangeant à froid (% en poids) :
- MARCOL 82⁽¹³⁾ : 5,0%
- agent épaississant de l'exemple 1 : 1,5 %
- NaOH: 10 %
- eau q.s. 100 %

⁽¹³⁾ : huile de paraffine commercialisée par la société ESSO.

La viscosité de cette composition est de 10 Pa.s et son pH est de 12 (après ajustement) ; sa stabilité est de 2 semaines à 50°C et de 3 mois à 40°C.

### Exemple 11 :

On prépare un fond de teint fluide en mélangeant (% en poids) :
- SIMULSOL 165⁽¹⁴⁾ : 5,0 %
- LANOL 99: 5,0 %
- LANOL 84D⁽¹⁵⁾: 8,0 %
- agent épaississant de l'exemple 1 : 1,2 %
- pigments et charges minérales : 10,0 %
- eau q.s. 100 %

(14) ; stéarate de glycérol auto-émulsionnable commercialisé par la société SEPPIC
(15) : dioctylmalate commercialisé par la société SEPPIC.

A ce mélange, on ajoute un conservateur et un parfum.

La viscosité de cette composition est de 20 Pa.s et son pH est de 6 (après ajustement) ; sa stabilité est de 1 mois à 50°C et de 3 mois à 40°C.

### Exemple 12 :

On prépare un lait solaire en mélangeant (% en poids) :
- MONTANOV 94 : 3,5 %
- LANOL 37T 10,0 %
- PARSOL NOX⁽¹⁶⁾: 5,0 %
- EUSOLEX 4360⁽¹⁷⁾: 2,0 %
- agent épaississant de l'exemple 1 : 1,8 %
- eau q.s. 100 %

⁽¹⁶⁾ : filtre solaire commercialisé par la société GIVAUDAN
⁽¹⁷⁾ : filtre solaire commercialisé par la société MERCK.

A ce mélange, on ajoute un conservateur et un parfum.

La viscosité de cette composition est de 18 Pa.s et son pH est de 7 (après ajustement) ; sa stabilité est de 1 mois à 50°C et de 3 mois à 40°C.

### Exemple 13 :

On prépare un gel contour des yeux en mélangeant (% en poids)
- agent épaississant de l'exemple 1 : 2,0 %
- pyrrolidone carboxylate de sodium : 0,2 %
- cyclométhicone⁽¹⁸⁾ : 2,0 %
- eau q.s. 100%

⁽¹⁸⁾ : Dow Corning 245 fluid, commercialisé par la société DOW CORNING.

A ce mélange, on ajoute un conservateur et un parfum.

La viscosité de cette composition est de 15 Pa.s et son pH est de 6 (après ajustement) ; sa stabilité est de 2 semaines à 50°C et de 3 mois à 40°C.

## Revendications

1. Agent épaississant consistant en une émulsion inverse eau dans huile, dans laquelle la phase aqueuse comprend un copolymère ayant des motifs dérivés (i) de l'acide acrylique sous forme libre ou sous forme de sel de sodium et (ii) d'un monomère monoacrylamide, **caractérisé en ce que** :
a) La phase huile est constituée d'un mélange dans un rapport pondéral compris entre 70/30 et 30/70, d'au moins une huile volatile choisie parmi les isoparaffines et d'au moins une huile non volatile choisie parmi les huiles de paraffine, les esters comportant plus de 10 atomes de carbone et, de préférence, de 14 à 34 atomes de carbone, formés par la condensation entre un acide gras et un mono alcool, et
b) le rapport molaire entre le monomère mono carboxylique et le monomère acrylamide, est compris entre 85/15 et 15/85 et de préférence entre 60/40 et 40/60.

2. Agent épaississant tel que défini à la revendication 1, **caractérisé en ce que** le copolymère comporte en outre un monomère poly fonctionnel, a une teneur comprise entre 0,1 et 0,2 milli-équivalents / mole des autres monomères.

3. Agent épaississant tel que défini à la revendication 2, **caractérisé en ce que** le monomère polyfonctionnel, est le méthylène bis-acrylamide.

4. Agent épaississant tel que défini à l'une des revendications 1 à 3, **caractérisé en ce qu'**il comprend en outre, au moins un tensioactif de préférence un agent tensioactif non-ionique, dont la HLB est supérieure ou égale à 8, de préférence supérieure ou égale à 10.

5. Agent épaississant tel que défini à la revendication 4, dans lequel l'agent tensioactif non-ionique est choisi parmi les alcools gras polyéthoxylés, les alkylphénols polyéthylènes, les esters de sorbitan, les esters de sorbitan polyéthoxylés, les alkylolamides ou les alkylpolyglycosides.

6. Agent épaississant tel que défini à l'une des revendications 1 à 5, dans lequel la phase huile est constituée d'un mélange, d'une huile d'isoparaffine, et d'une huile de paraffine.

7. Agent épaississant tel que défini à l'une des revendications 1 à 5, dans lequel la phase huile est constituée d'un mélange, d'une huile d'isoparaffine, et de stéaryl isostéarate.

8. Agent épaississant tel que défini à l'une des revendications 1 à 5, dans lequel la phase huile est constituée d'un mélange, d'une huile d'isoparaffine, et de cétéaryl octanoate.

9. Utilisation de l'agent épaississant, tel que défini à l'une des revendications 1 à 8, pour la préparation d 'une composition topique.

10. Composition topique sous forme d'une émulsion huile dans eau comprenant à titre d'agent épaississant, de 0,1% à 10% en poids de l'agent épaississant, tel que défini à l'une des revendications 1 à 8.

## Patentansprüche

1. Verdicker aus einer umgekehrten Wasser-in-Öl-Emulsion, in der die Wasserphase ein Copolymer mit (i) von Acrylsäure in freier Form oder in Form des Natriumsalzes und (ii) von einem Monoacrylamid-Monomer abgeleiteten Einheiten enthält, **dadurch gekennzeichnet, daß**:
a) die Ölphase sich aus einem Gemisch aus mindestens einem unter Isoparaffinölen ausgewählten flüchtigen Öl und mindestens einem unter Paraffinölen und durch Kondensation einer Fettsäure mit einem Monoalkohol erhaltenen Estern mit mehr als 10 Kohlenstoffatomen, vorzugsweise 14 bis 34 Kohlenstoffatomen ausgewählten nichtflüchtigen Öl in einem Gewichtsverhältnis zwischen 70/30 und 30/70 zusammensetzt und
b) das Molverhältnis zwischen dem Monocarbonsäure-Monomer und dem Acrylamid-Monomer zwischen 85/15 und 15/85, vorzugsweise zwischen 60/40 und 40/60 liegt.

2. Verdicker nach Anspruch 1, **dadurch gekennzeichnet, daß** das Copolymer außerdem auch noch ein polyfunktionelles Monomer in einer Menge zwischen 0,1 und 0,2 Milliäquivalenten/mol der anderen Monomere enthält.

3. Verdicker nach Anspruch 2, **dadurch gekennzeichnet, daß** es sich bei dem polyfunktionellen Monomer um Methylenbisacrylamid handelt.

4. Verdicker nach einem der Ansprüche 1 bis 3, **dadurch gekennzeichnet, daß** er außerdem auch noch mindestens ein Tensid vorzugsweise ein nichtionisches Tensid, mit einem HLB-Wert größer gleich 8, vorzugsweise größer gleich 10, enthält.

5. Verdicker nach Anspruch 4, **dadurch gekennzeichnet, daß** das nichtionische Tensid unter polyethoxylierten Fettalkoholen, polyethoxylierten Alkylphenolen, Sorbitanestern, polyethoxylierten Sorbitanestern, Alkylolamiden und Alkylpolyglycosiden ausgewählt ist.

6. Verdicker nach einem der Ansprüche 1 bis 5, **dadurch gekennzeichnet, daß** sich die Ölphase aus einem Gemisch aus einem Isoparaffinöl und einem Paraffinöl zusammensetzt.

7. Verdicker nach einem der Ansprüche 1 bis 5, **dadurch gekennzeichnet, daß** sich die Ölphase aus einem Gemisch aus einem Isoparaffinöl und Stearylisostearat zusammensetzt.

8. Verdicker nach einem der Ansprüche 1 bis 5, **dadurch gekennzeichnet, daß** sich die Ölphase aus einem Gemisch aus einem Isoparaffinöl und Cetearyloctanoat zusammensetzt.

9. Verwendung des Verdickers nach einem der Ansprüche 1 bis 8 zur Herstellung einer topischen Zusammensetzung.

10. Topische Zusammensetzung in Form einer Öl-in-Wasser-Emulsion, die als Verdicker 0,1 bis 10 Gew.-% Verdicker nach einem der Ansprüche 1 bis 8 enthält.

## Claims

1. Thickener consisting of a water-in-oil inverse emulsion, in which the aqueous phase comprises a copolymer containing units derived (i) from acrylic acid in free form or in the form of a sodium salt and (ii) from a monoacrylamide monomer, **characterized in that**:
a) the oil phase consists of a mixture, in a weight ratio of between 70/30 and 30/70, of at least one volatile oil chosen from isoparaffins and of at least one non-volatile oil chosen from liquid paraffins and esters containing more than 10 carbon atoms and preferably from 14 to 34 carbon atoms, formed by condensation between a fatty acid and a monoalcohol, and
b) the molar ratio between the monocarboxylic monomer and the acrylamide monomer is between 85/15 and 15/85 and preferably between 60/40 and 40/60.

2. Thickener as defined in Claim 1, **characterized in that** the copolymer also comprises a polyfunctional monomer, in a content of between 0.1 and 0.2 milli-equivalents/mol of the other monomers.

3. Thickener as defined in Claim 2, **characterized in that** the polyfunctional monomer is methylenebisacrylamide.

4. Thickener as defined in one of Claims 1 to 3, **characterized in that** it also comprises at least one surfactant, preferably a nonionic surfactant, whose HLB value is greater than or equal to 8 and preferably greater than or equal to 10.

5. Thickener as defined in Claim 4, in which the nonionic surfactant is chosen from polyethoxylated fatty alcohols, polyethoxylated alkylphenols, sorbitan esters, polyethoxylated sorbitan esters, alkylolamides and alkylpolyglycosides.

6. Thickener as defined in one of Claims 1 to 5, in which the oil phase consists of a mixture of a liquid isoparaffin and a liquid paraffin.

7. Thickener as defined in one of Claims 1 to 5, in which the oil phase consists of a mixture of a liquid isoparaffin and stearyl isostearate.

8. Thickener as defined in one of Claims 1 to 5, in which the oil phase consists of a mixture of a liquid isoparaffin and cetearyl octanoate.

9. Use of the thickener as defined in one of Claims 1 to 8 for the preparation of a topical composition.

10. Topical composition in the form of an oil-in-water emulsion comprising, as thickener, from 0.1% to 10% by weight of the thickener as defined in one of Claims 1 to 8.
